**Europäisches Patentamt**

**European Patent Office**

**Office européen des brevets**

(19)

(11) Veröffentlichungsnummer: **0 265 602**
**B1**

(12) **EUROPÄISCHE PATENTSCHRIFT**

(45) Veröffentlichungstag der Patentschrift:
**27.12.90**

(51) Int. Cl.⁵: **G01N 33/49**, G01N 27/06

(21) Anmeldenummer: **87111147.2**

(22) Anmeldetag: **01.08.87**

(54) Verfahren zur Bestimmung des Hämatokrit aus Vollblut sowie Vorrichtung zur Durchführung des Verfahrens.

(30) Priorität: **16.08.86 DE 3627814**

(43) Veröffentlichungstag der Anmeldung:
**04.05.88 Patentblatt 88/18**

(45) Bekanntmachung des Hinweises auf die Patenterteilung:
**27.12.90 Patentblatt 90/52**

(84) Benannte Vertragsstaaten:
**DE ES FR GB IT**

(56) Entgegenhaltungen:
**US-A- 3 997 838**
**US-A- 4 484 135**

**IEEE TRANSACTIONS ON BIOMEDICAL ENGINEERING, Band BME-30, Nr. 3, März 1983, Seiten 141-154, New York, US; E.A. TRAUTMAN et al.: "A practical analysis of the electrical conductivity of blood"**
**ANALYTICAL CHEMISTRY, Band 57, Nr. 3, März 1985, Seiten 578-580, Easton, Pennsylvania, US; S. DÜTSCH et al.: "Microprocessor-controlled ex vivo monitoring of sodium and potassium concentrations in undiluted urine with ion-selective electrodes"**
**PATENT ABSTRACTS OF JAPAN, Band 6, Nr. 125 (P-127), 10. Juli 1982; & JP - A - 57 50659 (YATORON K.K.) 25.03.1982**

(73) Patentinhaber: **Fresenius AG, Gluckensteinweg 5, D-6380 Bad Homburg v.d.H.(DE)**

(72) Erfinder: **Polaschegg, Hans-Dietrich, Dr., Grünwiesenweg 9, D-6370 Oberursel(DE)**
Erfinder: **Westphal, Detlef, Dr., Starenweg 1, D-6370 Oberursel(DE)**
Erfinder: **Metzner, Klaus,Dipl.-Phys., Alolfstrasse 17b, D-6380 Bad Homburg v.d.H.(DE)**

(74) Vertreter: **Dr. Fuchs, Dr. Luderschmidt Dipl.-Phys. Seids, Dr. Mehler Patentanwälte, Abraham-Lincoln-Strasse 7, D-6200 Wiesbaden(DE)**

**Beschreibung**

Die Erfindung betrifft ein Verfahren zur Bestimmung des Hämatokrit aus Vollblut, bei dem man die Leitfähigkeit von Vollblut und die Leitfähigkeit von Blutplasma bestimmt und aus den gewonnenen Leitfähigkeitswerten den Hamatokrit bestimmt.

Die Erfindung betrifft weiterhin eine Vorrichtung zur Bestimmung des Hamatokrit mit einer Einrichtung zur Bestimmung der Leitfähigkeit von Vollblut, einer Einrichtung zur Bestimm ung der Leitfähigkeit in Plasmawasser und einer Einheit zur Meßwertregistrierung.

Der Hamatokrit ist ein wichtiger physiologischer Parameter, der zur Beurteilung des Zustandes eines Patienten in der Allgemeinmedizin, vor allem aber im Bereich der Intensivmedizin, beispielsweise bei Operationen am offenen Herzen, sowie in der Dialysebehandlung herangezogen wird.

Zu Methoden für die Bestimmung des Hämatokrit gehört die Leitfähigkeitsmessung. Eine Vorrichtung zur Bestimmung des Hämatokritwerts von Blut auf Basis der Leitfähigkeitsmessung ist beispielsweise in der US-PS 4,547,735 sowie den darin genannten Druckschriften oder Biomed. Techn., Band 27 (1982), S. 171-175 beschrieben. Dabei wird eine Blutprobe zwischen zwei Elektroden hinsichtlich ihrer Leitfähigkeit vermessen, wobei die Sedimentation der Blutprobe im wesentlichen zu eliminieren ist.

Eine derartige Meßanordnung setzt eine Kalibrierungskurve voraus, mit der der gemessene Leitfähigkeitswert verglichen wird.

Darüberhinaus wird bei der bekannten Vorrichtung die absolute Leitfähigkeit, nicht jedoch die spezifische Leitfähigkeit gemessen, so daß die Konstanz der Zellenkonstante Voraussetzung ist. Im übrigen ist bei diesem Verfahren eine Temperaturstabilisierung zwingend notwendig.

Der Einsatz derartiger stationärer Vorrichtungen, bei denen eine dem Patienten entnommene Blutprobe zwischen zwei Elektroden plaziert wird, ist jedoch dann problematisch, wenn Blutproben vom Patienten mit unterschiedlichen Elektrolytspiegeln vermessen werden sollen. Die Leitfähigkeit im Vollblut wird namlich nicht nur vom Hämatokrit, sondern wesentlich auch von der Leitfähigkeit des den Strom leitenden Mediums, des Blutwassers bzw. Plasmas bestimmt.

Diese wiederum ist stark korreliert mit dem Elektrolytgehalt, d. h. abhängig von den Konzentrationen der hauptsächlich die Leitfähigkeit bestimmenden Ionenarten.

So ändert sich die Plasmaleitfähigkeit bei einer Variation der Natriumionenkonzentration von 130 auf 150 mmol/1 bei konst. Kalium von 4 mmol/1 um ca. 15%. Anhand einer bei 130 mmol/1 Natrium bestimmten Kalibrierungskurve würde ein Hkt von 30% nurmehr mit 22% - 23% Hkt gemessen.

In Fällen einer Elektrolytstörung wären nach dem oben beschriebenen Verfahren patientenspezifische Eichkurven erforderlich. Das Verfahren versagt gänzlich, wenn sich der Elektrolytspiegel während der Behandlung ändert, wie z.B. bei der Dialyse oder bei der Infusion hyper- bzw. hypoosmolarer Flüssigkeiten.

Zur Bestimmung des absoluten Hamatokrit bei der extrakorporalen Dialyse ist in der US-PS 4,484,135 ein Verfahren beschrieben, bei dem jeweils der Leitfähigkeitswert von Vollblut und von Plasmawasser bestimmt wird. Dabei wird das Plasmawasser durch Ultrafiltration aus dem Vollblut gewonnen und anschließend der Leitfähigkeitsmessung unterzogen.

Dabei konnte festgestellt werden, daß Änderungen des Hamatokrit mit den Änderungen des Blutvolumens korreliert sind, sofern das Erythrozytenvolumen insgesamt konstant bleibt.

Diese bekannte Vorrichtung weist jedoch den Nachteil auf, daß sie, bedingt durch die Notwendigkeit, das Plasmawasser erst durch Filtration gewinnen zu müssen, sehr aufwendig ist. Desweiteren führt die Hämofiltration aufgrund des bekannten Gibbs-Donnan-Effekts zu einer Verschiebung der Ionenkonzentrationen in Abhängigkeit vom Proteingehalt. Die solchermaßen veränderte Leitfähigkeit kann zu geringfügigen Fehlbestimmungen des Hämatokrits von wenigen %-Punkten führen.

Schließlich ist aus der FR-PS 2308099 ein Verfahren zur Bestimmung des Hämatokrit bekannt, bei dem einer quantitav erfaßten Blutprobe eine bestimmte Menge Elektolytlösung zugesetzt wird, wobei der zu bestimmende Elektrolyt der Blutprobe und der Elektrolyt der Elektrolytlösung identisch sind. Bei diesem Verfahren werden die Ausgangskonzentrationen und die Mischungskonzentration der Elektrolyte bestimmt, wobei anhand der sich ergebenden Werte der Hämatokrit errechnet werden kann.

Der Erfindung liegt die Aufgabe zugrunde, ein Verfahren zur Verfügung zu stellen, mit dem im Vollblut neben der Vollblutleitfähigkeit auch die Plasmaleitfähigkeit und somit der absolute Hämatokrit bestimmt werden kann.

Die Lösung der Aufgabe erfolgt durch die kennzeichnenden Merkmale des Anspruchs 1 bzw. 9.

Das erfindungsgemäße Verfahren weist zunächst den Vorteil auf, daß Vollblut direkt sowohl zur Bestimmung des Leitfähigkeitswerts von Vollblut als auch zur Bestimmung des Leitfähigkeitswerts von Plasmawasser eingesetzt werden kann.

Dabei wird die Leitfähigkeit des Plasmawassers im wesentlichen durch das Natriumion oder das Chloridion gebildet, wobei das Natriumion beispielsweise zu etwa 97% den Leitfähigkeitswert bildet und das Kaliumion sowie die übrigen Kationen den Restwert von etwa 3% darstellen. Diese Betrachtungsweise bezieht sich im wesentlichen auf die hier behandelten Änderungen der Leitfähigkeit im physiologischen Bereich. Wenn man den Leitfähigkeisbeitrag der Bicarbonat-Anionen als im wesentlichen konstant ansieht (Änderungen der Bicarbonat-konzentration von ± 4 mmol/1 führen zu einer Verfälschung des Hämatokrit von max. 1%-Punkt), ist die Änderung der Kationen korreliert mit einer entsprechenden Änderung der Chlorid-Anionen. Ein selektiver Einfluß der Anionen muß daher in erster Näherung nicht betrachtet zu werden.

Der Einsatz von ionenselektiven Elektroden ist insofern vorteilhaft, als durch diese Elektroden die Konzentration der Elektrolyte bestimmt wird, die von dem Erythrozytenvolumen unabhängig ist. Die sich hieraus ergebende Konzentration kann mit der spezifischen Leitfähigkeit des spezifischen Ions multipliziert zum Leitfähigkeitswert des Ions umgerechnet werden, woraus sich die Gesamtleitfähigkeit des Plasmawassers durch entsprechende Extrapolation errechnen läßt.

In einer weiteren Ausführungsform kann zusätzlich zu der natriumionenselektiven Elektrode eine kaliumionenselektive Elektrode eingesetzt werden, um die Gesamtelektrolytleitfähigkeit im Plasma exakter, d.h. zu mindestens 99% zu bestimmen, wobei der unbestimmte Elektrolytanteil, der im wesentlichen auf Magnesium- und Calciumionen zurückzuführen ist, ohne signifikanten Fehler als konstant für die Gesamtleitfähigkeit angesehen werden kann.

Diese ionenselektiven Elektroden werden vorteilhafterweise in einer einzigen Vorrichtung zusammen mit einer Leitfähigkeitsmeßeinrichtung vorgesehen, so daß sowohl die in einer Blutprobe enthaltenen Elektrolyte als auch die Leitfähigkeit der Blutprobe nebeneinander bestimmt werden können, ohne daß die Abtrennung von Plasmawasser aus dem Vollblut mittels eines Hämofilters notwendig wäre.

Besonders vorteilhaft wird eine Durchflußeinrichtung eingesetzt, die einen Durchflußkanal aufweist, in den die ionenselektiven Elektroden und die Leitfähigkeitsmeßzelle eingeschaltet sind. In einen solchen Durchflußkanal kann mit Hilfe einer Spritze die Blutprobe eingespritzt werden oder aber das Ende des Durchflußkanals kann mit einer blutführenden extrakorporalen Leitung verbunden sein, auf die eine Pumpe einwirkt, wodurch Blut kontinuierlich durch die Durchflußmeßeinrichtung gepump t wird. So kann einem extrakorporalen Blutkreislauf, der beispielsweise bei der Hämodialyse aufgebaut wird, kontinuierlich mit Hilfe einer abzweigenden Leitung Blut entzogen werden, das in einem derartigen Durchflußanalysator analysiert wird. Des weiteren kann eine derartige Blutprobe aber auch über einen Dauerkatheter entnommen werden, der in ein Körpergefäß eingeführt worden ist. Diese Methode kann vorteilhafterweise zur Überwachung von Patienten auf einer Intensivstation eingesetzt werden.

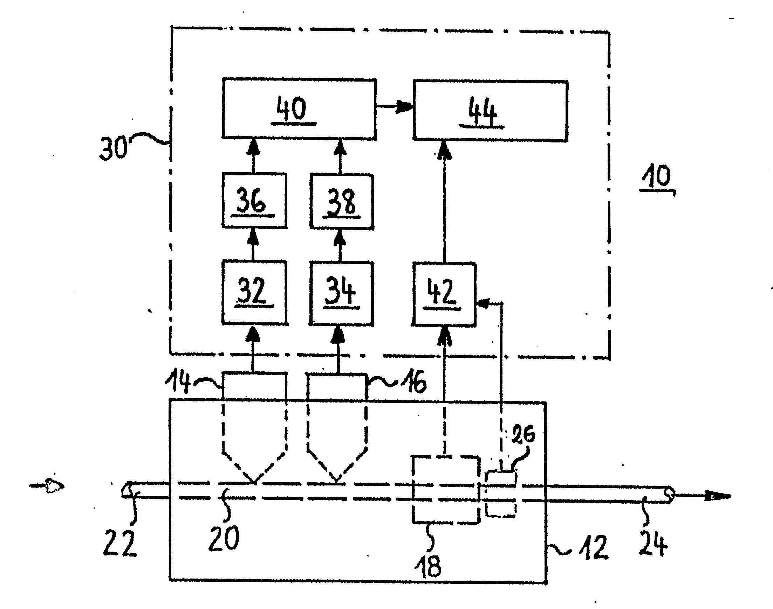

Weitere Vorteile, Merkmale und Einzelheiten werden anhand eines Ausführungsbeispiels unter Bezug auf die Zeichnung erläutert.

Es zeigt die Figur eine Prinzipskizze einer Durchflußanordnung mit zwei ionenselektiven Elektroden und einer Leitfähigkeitszelle gemäß der Erfindung.

In der Figur ist die Vorrichtung zur Bestimmung des Hämatokrit mit 10 bezeichnet. Diese Vorrichtung 10 weist ein Gehäuse 12 auf, in dem eine natriumionenselektive Elektrode 14, eine kaliumionenselektive Elektrode 16 und eine Leitfähigkeitszelle 18 untergebracht sind, wobei die Elektroden 14 und 16 in an sich bekannter Weise mit einer nichtgezeigten Referenzelektrodenanordnung verbunden sind. Des weiteren weist das Gehäuse 12 einen Durchflußkanal 20 auf, der mit einem Eingangsstutzen 22 und einem Ausgangsstutzen 24 in Verbindung ist.

Mit diesem Durchflußkanal 20 sind die natriumionenselektive Elektrode 14, die kaliumionenselektive Elektrode 16 und die LF-Zelle 18 derart verbunden, daß das durch den Durchflußkanal 20 zu führende Blut mit ihnen in Berührung kommen und somit jeweils ein Meßsignal auslösen kann.

Derartige Durchflußanordnungen sind beispielsweise aus der DE-OS 34 16 956 bekannt, auf deren Offenbarung ausdrücklich Bezug genommen wird. Insofern kann auf weitere Einzelheiten der ionenselektiven Elektroden, die im übrigen bekannt sind, verzichtet werden. Schließlich sind Leitfähigkeitsmeßzellen ebenfalls bekannt und müssen daher nicht im einzelnen erläutert werden.

Blut kann mit Hilfe einer nichtgezeigten Zuführungseinrichtung dem Durchflußkanal 20 zugeführt und durch diesen gefördert werden. Zur Messung verbleibt die Blutprobe stationär im Durchflußkanal 20 und wird anschließend in einem Spülvorgang aus dem Durchflußkanal mit einem Spülfluid (Luft, Kochsalzlösung oder dgl.) verdrängt.

Das Gehäuse 12 kann weiterhin eine nichtgezeigte Thermostateinrichtung oder eine am Durchflußkanal angeordnete Temperaturmeßeinrichtung 26 aufweisen, um Temperatureffekte durch Stabilisierung bzw. rechnerisch zu kompensieren. Somit kann auch die Vorrichtung 10 thermisch mit der Umgebung im Gleichgewicht sein, ohne daß eine Thermostatisierung notwendig wäre.

Die natriumionenselektive Elektrode 14, die kaliumionenselektive Elektrode 16 und die Leitfähigkeitszelle 18 sind mit einem Rechner 30 verbunden, der strichliert in der Figur dargestellt ist. Dieser Rechner 30 weist die nachstehend beschriebenen Einheiten auf, mit denen der Hämatokrit aus den von den Elektroden und der Leitfähigkeitszelle abgegebenen Signalen errechnet werden kann.

In der Recheneinheit 32 bzw. 34 wird zunächst aus dem Signal der natriumionenselektiven Elektrode 14 bzw. der kaliumselektiven Elektrode 16 die Natriumionenkonzentration $c_{Na}$ bzw. die Kaliumionenkonzentration $c_K$ errechnet. Hierzu werden die ionenselektiven Elektroden nach einem bekannten Verfahren mit Normlösungen kalibriert, wobei die Recheneinheiten 32 und 34 zur Errechnung der Ionenkonzentrationen die gemessenen Signale mit Referenzsignalen vergleichen und somit die tatsächliche, im Blut enthaltene Ionenkonzentration ermitteln.

Den in den Recheneinheiten 32 und 34 ermittelten Konzentrationswerten wird in den Zuordnungseinheiten 36 und 38 eine empirische Konstante A bzw. B zugeordnet, so daß sich hieraus für die Natriumionenkonzentrationen das Produkt $A * c_{Na}$ und für die Kaliumionenkonzentrationen das Produkt $B * c_K$ ergeben. Die empirischen Größen A und B werden zuvor experimentell bestimmt und stellen somit Fitgrößen dar. Sie enthalten im wesentlichen die Leitfähigkeitskonstanten der Natrium- bzw. Kaliumionen im entsprechenden Konzentrationsbereich.

Die in den Zuordnungseinheiten 36 und 38 ermittelten Produkte stellen somit die Leitfähigkeit der jeweiligen Natrium- und Kaliumionen im Plasmawasser dar, zu denen zusätzlich noch eine Konstante D hinzuzufügen ist, die die restlichen im Blut enthaltenen Magnesium- und Calciumionen berücksichtigt. Hierzu werden die in den Einheiten 36 und 38 ermittelten Werte in einem Summierer 40 zusammen mit der Konstante D zu der Plasmaleitfähigkeit LF $_P$ addiert, wie dies aus Gleichung (1) ersichtlich ist.

(1) $LF_P = A * c_{Na} + E$
(2) $LF_P = A * c_{Na} + B * c_K + D$

wobei demzufolge $E = B * c_k + D$ ist.

Die Leitfähigkeitsmeßzelle 18 und ggf. die Temperaturmeßeinrichtung 26 sind mit einer Einheit 42 zur Bestimmung der Leitfähigkeit von Vollblut LFVB verbunden, wobei der ermittelte Wert ggf. temperaturkompensiert werden kann. Diese Einheit 42 errechnet aus den übertragenen Signalen diesen Leitfähigkeitswert und gibt den ermittelten Wert an eine Einheit 44 weiter, in der aus folgender Gleichung (3)

$$Hkt = K * \left( 1 - \frac{LF_{VB}}{LF_P} \right) = K * N \qquad (3)$$

der Hämatokrit Hkt ermittelt wird, wobei die Konstante K eine empirische Fitkonstante ist. Überlicherweise werden samtiche vorstehend genannten Konstanten K, A, B, D oder E anhand einer Korrelationsmeßung mit bekannten Plasmaleitfähigkeitswerten normiert, die sich beispielsweise aus der Hämatokritmeßung in der Zentrifuge ergeben.

Diese Einheit 44 wird weiterhin mit dem in dem Summierer 40 ermittelten Leitfähigkeitswert gespeist und ermittelt so nach der vorstehenden Gleichung den Hämatokrit.

An diese Einheit ist eine übliche, nicht gezeigte Anzeige oder eine sonstige Einrichtung zur Speicherung des ermittelten Werts angeschlossen.

Es hat sich gezeigt, daß der mit dieser Vorrichtung ermittelte Hämatokrit in Übereinstimmung steht mit dem durch Zentrifugation gemessenen Hämatokrit. Mit ionenselektiven Elektroden ist es möglich, die Aktivität von Ionen und durch einfache Umrechnung die Konzentration von Ionen in der wäßrigen Phase des Bluts zu bestimmen. Aus den im Vollblut mit Hilfe von ionenselektiven Elektroden bestimmten Ionenkonzentrationen kann man somit die Leitfähigkeit von Plasmawasser errechnen. Mit Hilfe dieses ermittelten Plasmaleitfähigkeitswerts und des Vollblutleitfähigkeitswerts läßt sich dann - wie oben erläutert - der Hämatokrit errechnen.

Wie bereits vorstehend erwähnt, können anstelle von kationenselektiven Elektroden auch anionenselektive Elektroden, beispielsweise eine chlorid- und ggf. eine bicarbonatselektive Elektrode eingesetzt werden.

Die oben genannte Beziehung für Hkt kann nur für einen relativ engen Hämatokritbereich angepaßt werden. Vorteilhaft ist es, die Größe Hkt in einer quadratischen oder kubischen Approximation zu ermitteln, um die bekannten Nichtlinearitäten in der Hämatokrit-Leitfähigkeitsbeziehung mitzuerfassen. Dadurch kann der Hämatokrit im gesammten Bereich von ca. 15% - 65% bestimmt werden.

Hierzu wird die Einheit 36 bzw. der Summierer 40 mit entsprechenden Konstanten versehen, die in Verbindung mit den Ionenkonzentrationen zum Plasmaleitfähigkeitswert führen.

In gleicher Weise wie die Natriumkonzentration kann mit einer chloridionenselektiven elektrode der Chloridgehalt von Blut bestimmt werden, aus dem sich entsprechend der Gleichung (4)

$LF_P = F * c_{Cl} + G \quad (4)$

der Hämatokrit berechnet, wobei die Konstanten F und G wiederum wie die vorstehend genannten Konstanten K etc. empirische Konstanten sind, die durch Vergleich mit an sich bekannten Plasmaleitfähigkeitswerten bzw. Hämatokritwerten ermittelt werden.

Desgleichen kann die Konstante K – wie vorstehend erwähnt - anhand von Blutproben mit bekannten Hämatokritwerten ermittelt werden.

Es hat sich nunmehr herausgestellt, daß brauchbare Hämatokritwerte mit folgenden Werten erhalten werden, wobei die Vollblutleitfähigkeit in $mS/cm^2$ und die Konzentrationen in $mmol/1$ gemessen werden.

Die Konstante K liegt in einem Bereich von 72–88, vorzugsweise etwa 80, die Konstante A in einem Bereich von etwa 0,058–0,082, vorzugsweise etwa 0,07, die Konstante B bei etwa 1,2 * A und die Konstante D in einem Bereich von 0–2, vorzugsweise etwa 1. Die mit diesen Werten ermittelten Hämatokritwerte stimmen mit den Werten gemäß den üblichen Methoden zur Bestimmung des Hämatokrit, die untereinander ebenfalls unterschiedliche Werte ergeben, innerhalb von etwa 2– 3 Hämatokritgraden überein,
wobei diese Konstanten besonders vorteilhaft in einem Hämatokritbereich von etwa 18-48 einsetzbar
sind.

Neben dieser linearen Approximation wird vorteilhafterweise eine quadratische Approximation nach
folgender Gleichung (5) durchgeführt:

$$Hkt = P * N^2 + Q * N \quad (5)$$

wobei die Werte P und Q empirisch anhand vorstehender Rechenmethode ermittelt wurden und N den in
Gleichung (3) angegebenen Klammerausdruck angibt. Dabei liegt die Konstante P zwischen etwa 37-57,
vorzugsweise bei etwa 47 und die Konstante Q zwischen 44-60, vorzugsweise bei etwa 52.

Mit der quadratischen Approximation kann der Hkt-Meßbereich ausgeweitet werden auf über 60 und
bis unterhalb 15, wobei die vorstehenden, bei der linearen Approximation genannten Abweichungen eingehalten werden können.

## Patentansprüche

1. Verfahren zur Bestimmung des Hämatokrit aus Vollblut, bei dem man die Leitfähigkeit von Vollblut
und die Leitfähigkeit von Blutplasma bestimmt und aus den gewonnenen Leitfähigkeitswerten den Hämatokrit ermittelt, dadurch gekennzeichnet, daß man die im Blut vorliegende Natriumionenkonzentration $c_{Na}$
oder Chloridionenkonzentration $c_{Cl}$ bestimmt und aus dem so erhaltenen Konzentrationswert die Plasmaleitfähigkeit gemäß der Gleichung

a) $LF_P = A * c_{Na} + E$ oder

b) $LF_P = F * c_{Cl} + G$

ermittelt, wobei die Konstanten A, E, F und G mittels eines an sich bekannten Hämatokritmeßverfahrens zuvor ermittelt werden.

2. Verfahren nach Anspruch 1, dadurch gekennzeichnet, daß man neben der Natriumionenkonzentration die Kaliumionenkonzentration $c_K$ bestimmt.

3. Verfahren nach Anspruch 2, dadurch gekennzeichnet, daß man den Leitfähigkeitswert von Blutplasma entsprechend der linearen Gleichung

$LF_P = A * c_{Na} + B * c_K + D$

errechnet, wobei B und D Konstanten sind, die entsprechend dem Verfahren von Anspruch 1 ermittelt
wurden.

4. Verfahren nach Anspruch 3, dadurch gekennzeichnet, daß die Konstante K 72–88, die Konstante
A 0,058–0,082, die Konstante B 1,2 * A und die Konstante D 0–2 betragen.

5. Verfahren nach Anspruch 4, dadurch gekennzeichnet, daß K 80, A 0,07 und D 1 sind.

6. Verfahren nach Anspruch 2, dadurch gekennzeichnet, daß man eine quadratische Approximation
entsprechend der Gleichung

$Hkt = P * N^2 + Q * N$,

durchführt, wobei $N = 1 – LF_{VB}/LF_P$ bedeutet, und $LF_P$ entsprechend der Gleichung von Anspruch 3
berechnet wird und P zwischen 37–57 und Q zwischen 44 und 60 liegt.

7. Verfahren nach Anspruch 6, dadurch gekennzeichnet, daß P 47 und Q 52 betragen.

8. Verfahren nach einem der Ansprüche 1 bis 7, dadurch gekennzeichnet, daß man die Bestimmung
der Ionenkonzentrationen mit ionenselektiven Elektroden durchführt.

9. Vorrichtung zur Bestimmung des Hämatokrit nach Anspruch 1 mit einer Einrichtung zur Bestimmung
der Leitfähigkeit von Vollblut, einer Einrichtung zur Ermittlung der Leitfähigkeit in Plasmawasser und einer Einheit zur Meßwertregistrierung, dadurch gekennzeichnet, daß die Einrichtung zur Ermittlung der
Leitfähigkeit in Plasmawasser eine natriumionenselektive Elektrode (14) oder eine chloridionenselektive
Elektrode, mit der die Konzentration von Natriumionen oder Chloridionen unmittelbar in Vollblut bestimmt
wird, und eine Recheneinheit zur Bestimmung der Plasmaleit- fähigkeit (32–40) aus dem Signal der natriumionen- selektiven Elektrode (14) oder der chloridionenselektiven Elektrode aufweist.

10. Vorrichtung nach Anspruch 9, dadurch gekennzeichnet, daß sich neben der natriumionenselektiven Elektrode eine kaliumionenselektive Elektrode (16) befindet.

11. Vorrichtung nach Anspruch 9, dadurch gekennzeichnet, daß die Recheneinheit eine Einheit (32,
34) zur Bestimmung der Natrium- und der Kaliumionenkonzentrationen aus dem Signal der natriumionenselektiven und kaliumionenselektiven Elektroden (14, 16), eine Zuordnungseinheit (36, 38), mit der die in
der Einheit (32, 34) ermittelten Konzentrationswerte jeweils mit einer Konstante A bzw. B multipliziert
werden, und einen Summierer (40) aufweist, mit der die in den Zuordnungseinheiten (36, 38) ermittelten
Werte zusammen mit einer weiteren Konstante D addiert werden.

12. Vorrichtung nach der Anspruch 9, dadurch gekennzeichnet, daß die Einrichtung zur Bestimmung
der Leitfähigkeit von Vollblut eine Leitfähigkeitszelle (18) und eine Einheit (42) zur Bestimmung des Leitfähigkeitswerts aus dem von der Leitfähigkeitszelle abgegebenen Signal aufweist.

13. Vorrichtung nach Anspruch 12, dadurch gekennzeichnet, daß der Rechner (30) eine Einheit (44) zur Ermittlung des Hämatokrit aus den vom Summierer (40) abgegebenen Plasmaleitfähigkeitswert und dem von der Einheit (42) abgegebenen Vollblutleitfähigkeitswert aufweist.

14. Vorrichtung nach Anspruch 10, dadurch gekennzeichnet, daß sich die ionenselektiven Elektroden (14, 16) und die Leitfähigkeitszelle (18) in einem einen Durchflußkanal (20) aufweisenden Gehäuse (12) befinden.

## Claims

1. A method to determine hematocrit of whole blood wherein the conductivity of whole blood and the conductivity of blood plasma are determined and the hematocrit is established from the obtained values of the conductivity, characterized in that the sodium concentration $c_{Na}$ or the chloric ion concentration $c_{Cl}$ existing in the blood is determined and that from the thus obtained values or the concentration the conductivity of the plasma is established according to the equation

a) $LF_P = A * c_{Na} + E$ or

b) $LF_P = F * c_{Cl} + G$

the constants A, E, F, and G being previously determined by a measuring method of hematocrit being well known in itself.

2. A method according to claim 1, characterized in that the potassium ion concentration $C_K$ is determined in addition to the sodium concentration.

3. A method according to claim 2, characterized in that the value of the conductivity of blood plasma is calculated in accordance with the linear equation

$LF_P = A * c_{Na} + B * C_K + D$

B and D being constants determined according to the method of claim 1.

4. A method according to claim 3, characterized in that the constant K amounts to 72–88, the constant A amounts to 0.058–0.082, the constant B amounts to 1.2 x A, and the constant D amounts to 0–2.

5. A method according to claim 4, characterized in that K equals 80, A equals 0.07, and D equals 1.

6. A method according to claim 2, characterized in that a quadratic approximation is performed according to the equation

$Hkt = P \times N^2 + Q \times N$

where N signifies $1 - LF_{VB}/LF_P$ and $LF_P$ is calculated in accordance with the equation of claim 3 and P is between 37–57 and Q is between 44 and 60.

7. A method according to claim 6, characterized in that P amounts to 47 and Q amounts to 52.

8. A method according to one of the claims 1–7, characterized in that the ion concentration is determined by means of an ion-selective electrode.

9. A device to determine hematocrit according to claim 1 comprising an equipment to determine the conductivity of whole blood, an equipment to determine the conductivity in plasma water, and a unit to record measured values, characterized in that the equipment for the determination of the conductivity in plasma water comprises a sodium-selective electrode (14) or a chloric ion-selective electrode with which the concentration of sodion or chloric ions in whole blood is immediately determined and an arithmetic unit to determine plasma conductivity (32–40) from the signal of the sodion-selective electrode (14) or the chloric ion-selective electrode.

10. A device according to claim 9, characterized in that a potassium ion-selective electrode (16) is positioned beside the sodion-selective electrode

11. A device according to claim 9, characterized in that the arithmetic unit comprises a unit (32, 34) to determine the sodion and the potassium ion concentrations from the signal of the sodion-selective and potassium ion-selective electrodes (14, 16), an assignment unit (36, 38) with which the values of concentration being determined in the unit (32, 34) are multiplied in each case with the constant A or B respectively, and a summer (40) with which the values determined in the assignment units (36, 38) are added with a further constant D.

12. A device according to claim 9, characterized in that the equipment for the determination of conductivity of whole blood comprises a conductivity cell (18) and a unit (42) to determine the conductivity of the signal being emitted from the conductivity cell.

13. A device according to claim 12, characterized in that the summer (30) comprises a unit (44) to determine hematocrit from the values of the plasma conductivity being emitted from the summer (40) and the value of the conductivity of the whole blood being emitted from the unit (42).

14. A device according to claim 10, characterized in that the ion-selective electrodes (14, 16) and the conductivity cell (18) are arranged in a case (12) comprising a flow-through channel (20).

## Revendications

1. Procédé pour la détermination de l'hématocrite du sang, selon lequel on détermine la conductibilité du sang et la conductibilité du plasma sanguin et l'on déduit l'hématocrite des valeurs de conductibilité obtenues, caractérisé en ce que l'on détermine la concentration en ions sodium $C_{Na}$ ou la concentration en

ions chlorure $C_{Cl}$ présente dans le sang et que l'on déduit de la valeur de concentration ainsi obtenue la valeur de conductibilité du plasma selon l'équation

a) $LF_P = A * C_{Na} + E$ ou

b) $LF_P = F * CCl + G$

sachant que les constantes A, E, F et G ont été déterminées auparavant au moyen d'un procédé de détermination de l'hématocrite conu en soi.

2. Procédé selon la revendication 1, caractérisé en ce que l'on détermine outre la concentration des ions sodium, la concentration en ions potassium $C_K$.

3. Procédé selon la revendication 2, caractérisé en ce que l'on calcule la valeur de conductibilité du plasma sanguin selon l'équation linéaire $LF_P = A * C_{Na} + B * c_K + D$

sachant que B et D sont des constantes qui ont été déterminées selon le procédé de la revendication 1.

4. Procédé selon la revendication 3, caractérisé en ce que la constante K se situe entre 72 et 88, la constante A entre 0,058 et 0,082, la constante B s'élève à 1,2 * A et la constante D se situe entre 0 et 2.

5. Procédé selon la revendication 4, caractérisé en ce que K est égal à 80, A à 0,07 et D à 1.

6. Procédé selon la revendication 2, caractérisé en ce que l'on procède à une approximation carrée conformément à l'équation

$Hkt = P * N^2 + Q* N$,

sachant que $N = 1-LF_{VB}/LF_P$, que $LF_P$ est calculé conformément à l'équation de la revendication 3 et que P se situe entre 37 et 57 et Q entre 44 et 60.

7. Procédé selon la revendication 6, caractérisé en ce que P est égal à 47 et Q à 52.

8. Procédé selon l'une des revendications 1 à 7, caractérisé en ce que l'on détermine les concentrations en ions à l'aide d'électrodes sélectives d'ions.

9. Dispositif pour la détermination de l'hématocrite selon la revendication 1 comportant un système de détermination de la conductibilité du sang, un système de détermination de la conductibilité de l'eau du plasma et une unité pour l'enregistrement des valeurs de mesure, caractérisé en ce que le système de détermination de la conductibilité de l'eau du plasma comporte une électrode sélective d'ions sodium (14) ou une électrode sélective d'ions chlorure, avec laquelle la concentration en ions sodium ou en ions chlorure est directement déterminée dans le sang, ainsi qu'une unité de calcul pour la détermination de la conductibilité du plasma (32–40) à partir du signal de l'électrode sélective d'ions sodium (14) ou de l'électrode sélective ions chlorure.

10. Dispositif selon la revendication 9, caractérisé en ce qu'une électrode sélective d'ions sodium se trouve à côté d'une électrode sélective d'ions potassium (16).

11. Dispositif selon la revendication 9, caractérisé en ce que l'unité de calcul comprend une unité (32, 34) pour la détermination des concentrations en ions sodium et potassium à partir des signaux des électrodes sélectives d'ions sodium et d'ions potassium (14, 16), une unité d'attribution (36, 38), au moyen de laquelle les valeurs de concentration calculées par l'unité (32, 34), sont respectivement multipliées par une constante A et B, ainsi qu'un additionneur (40), au moyen duquel les valeurs obtenues dans les unités (36, 38) sont additionnées à une constante supplémentaire D.

12. Dispositif selon la revendication 9, caractérisé en ce que le système de détermination de la conductibilité du sang comprend une cellule de mesure de conductibilité (18) et une unité (42) pour la détermination de la valeur de conductibilité à partir du signal émis par la cellule de mesure de conductibilité.

13. Dispositif selon la revendication 12, caractérisé en ce que le calculateur (30) comporte une unité (44) pour la détermination de l'hématocrite à partir de la valeur de conductibilité du plasma donnée par l'additionneur (40) et de la valeur de conductibilité du sang donnée par l'unité (42).

14. Dispositif selon la revendication 10, caractérisé en ce que les électrodes sélectives d'ions (14, 16) et la cellule de mesure de conductibilité (18) sont implantées dans un boîtier (12) pourvu d'un canal d'écoulement (20).